# EUROPÄISCHE PATENTSCHRIFT

(11) **EP 2 488 231 B1**
(45) Veröffentlichungstag und Bekanntmachung des Hinweises auf die Patenterteilung: **04.12.2013**
(21) Anmeldenummer: 10772969.1
(22) Anmeldetag: 11.10.2010
(51) Int. Cl.: A61M 5/168, A61M 5/172, A61M 1/36, A61M 5/142, A61M 5/44

(54) **VORRICHTUNG ZUR BEHANDLUNG EINES INDIVIDUUMS MIT HERZMINDERLEISTUNG, HERZSTILLSTAND, KREISLAUFSTILLSTAND ODER SCHLAGANFALL**
DEVICE FOR TREATING AN INDIVIDUAL SUFFERING FROM CARDIAC INSUFFICIENCY, CARDIAC ARREST, CIRCULATORY ARREST OR STROKE
DISPOSITIF DE TRAITEMENT D'UN PATIENT SOUFFRANT D'UNE INSUFFISANCE CARDIAQUE, D'UN ARRÊT CARDIAQUE, D'UN ARRÊT CIRCULATOIRE OU D'UN ACCIDENT VASCULAIRE CÉRÉBRAL

(30) Priorität: 12.10.2009 DE 102009045589
(43) Veröffentlichungstag der Anmeldung: 22.08.2012
(73) Patentinhaber: ResuSciTec GmbH, 79098 Freiburg (DE)
(72) Erfinder: BEYERSDORF, Friedhelm, 79104 Freiburg (DE)
(74) Vertreter: Rösler, Uwe
(86) Internationale Anmeldenummer: PCT/EP2010/006199
(87) Internationale Veröffentlichungsnummer: WO 2011/045011

(56) Entgegenhaltungen:
- WO-A2-2005/025646
- WO-A2-2006/091650
- US-A- 5 879 316

## Beschreibung

Die Erfindung bezieht sich auf eine Vorrichtung zur Behandlung eines Individuums mit Herzminderleistung, Herzstillstand, Kreislaufstillstand oder Schlaganfall.

Mit dem derzeitigen Kenntnisstand sowie den gängigen therapeutischen Verfahren lassen sich Individuen, insbesondere Patienten, die einen Herzstillstand erlitten haben, nur dann ohne Schädigungen des Gehirns sowie der Herzfunktionen wieder beleben, sofern eine kardiopulmonale Reanimation innerhalb eines Zeitraumes von drei bis fünf Minuten nach dem eingetretenen Herstillstand erfolgreich durchgeführt wird. Reanimationen, die mit weiterer zeitlicher Verzögerung durchgeführt werden, führen unweigerlich zu schweren zerebralen Schädigungen, die von der Reperfusion mit normalem Blut, das das ischämische Gewebe massiv irreversibel schädigt, herrühren.

Vornehmlich in der Herzchirurgie nehmen Ischämie- und Reperfusionsveränderungen in Gewebebereichen eine zentrale Rolle ein. So wird beispielsweise eine Myokardischämie entweder vom Chirurgen selbst induziert, beispielsweise im Rahmen einer globalen Ischämie durch Aortenabklemmung, oder auch bei einer Herztransplantation bzw. regional in der "Off-pump-Chirurgie" zur koronaren Bypass-Anlage. Darüber hinaus kommen aber auch Patienten mit Myokardischämie notfallmäßig zur Operation, die beispielsweise einen kardiogenen Schock, einen akuten Koronarverschluss oder einen Zustand unmittelbar nach einer Wiederbelebung einnehmen. Aus diesen Gründen haben sich Herzchirurgen bereits seit Jahrzehnten intensiv mit Ischämie- und Reperfusionsphänomenen beschäftigt. Nach dem heutigen Wissensstand kann davon ausgegangen werden, dass eine Ischämie, selbst eine lang andauernde Ischämie, nur relativ geringe strukturelle Schädigungen an dem Herzmuskel hervorruft. Wird jedoch nach einem derartigen ischämischen Insult das Myokard mit normalem Blut unter "physiologischen" Bedingungen reperfundiert, so setzt explosionsartig ein zusätzlicher Schädigungsmechanismus ein, der inzwischen als "Reperfusionsschaden" gut erforscht ist. Bei Reperfusion eines ischämisch geschädigten Myokards mit normalem Blut setzen schlagartig Prozesse ein, die das bereits geschädigte Gewebe endgültig zu zerstören vermögen.

### Stand der Technik

Zur Vermeidung bzw. vollständigen Verhinderung der durch Reperfusion mit normalem Blut auftretenden Reperfusionsschäden sind Konzepte entwickelt worden, die nach Revaskularisation zunächst eine Behandlung des ischämisch geschädigten Myokards anstreben, indem sowohl die Zusammensetzung des initialen Reperfusates als auch die Bedingungen der initialen Reperfusion darauf ausgerichtet sind, die während der Ischämie entstandenen Schäden zu therapieren bzw. möglicherweise auftretende Reperfusionsschäden von vorneherein auszuschließen.

Das Konzept der kontrollierten Reperfusion basiert zum einen darauf, das initiale Reperfusat abweichend vom körpereigenen Blut zu verändern sowie die Bedingungen der initialen Reperfusion zu modifizieren.

In diesem Zusammenhang geht aus der DE 696 31 046 T2 eine Vorrichtung zur Behandlung eines Patienten mit Herzstillstand hervor, die sich der bekannten Technik der sog. selektiven Aortenbogenperfusion, kurz SAAP, bedient, gemäß der zur Durchführung einer relativ isolierten Perfusion des Herzens und Gehirns bei einem Patienten ein Ballonoklusionskatheter zumeist über die Femoralarterie an den Ort des absteigenden Aortenbogens platziert und anschließend dilatiert wird und nachfolgend über das Lumen des SAAP-Katheters eine oxygenierte Blutersatzlösung, beispielsweise Perfluorokarbonemulsion oder eine polymerisierte Hämoglobinlösung infundiert wird. Die auch als Schutzlösung bezeichnete Blutersatzlösung wird mit Hilfe eines Impulsgabemittels unter pulsierendem Rhythmus intrakorporal verabreicht. In einer Ausführungsvariante verfügt die bekannte Vorrichtung über ein Blutentnahmemittel, mit dem dem Patienten Blut entnommen wird, das einem Blutoxygenierungsmittel zur Sauerstoffanreicherung zugeführt und gemeinsam mit einer dem oxygenierten Blut verabreichten Schutzlösung dem Patienten über ein Blutrückführungsmittel infundiert wird. Anzumerken ist in diesem Zusammenhang, dass zur Durchführung einer selektiven Aortenbogenperfusion und somit zur Anwendung der vorstehenden Vorrichtung ein chirurgischer Eingriff und damit verbunden eine klinische Infrastruktur erforderlich ist.

Auch aus der US 5,195,942 ist eine vergleichbare Vorgehensweise zur Wiederbelebung einer Person zu entnehmen, bei der durch Aufblasen eines Ballonkatheters im Bereich der aufsteigenden Aorta zum Zwecke der Blutflusserhöhung in die Koronararterien eine blutkompatible, sauerstoffreiche Flüssigkeit zur weiteren Durchströmung in die Koronararterien injiziert wird.

Die US 7,387,798 B2 beschreibt ein gattungsgemäßes Verfahren zur Wiederbelebung von Patienten mit Herzstillstand, bei dem dem Patienten eine Liquor-Flüssigkeit aus dem subarachnoiden Bereich des zentralen Nervensystems entnommen wird. Eine künstlich zusammengesetzte Hirn-Rückenmarksflüssigkeit, die eine Vielzahl Komponenten enthält, wie beispielsweise Natrium, Kalium, Kalzium, Magnesium, Wasser, Polypeptide, Insulin sowie ATP, wird anschließend infundiert, woraufhin eine konventionelle kardiopulmonale Reanimation erfolgt.

Aus der US 5,416,078 ist die technische Lehre zu entnehmen, zur Wiederbelebung eines Menschen bzw. Tieres eine Lösung aus Deferoxamine mit wasserlöslichen Biopolymeren dem zu behandelnden Patienten zu verabreichen.

In der WO 94/21195 wird beschrieben zur Organvorbereitung zum Schutze des Organs gegenüber Ischämieschädigungen dem jeweiligen Organ ein Agonist zu dem A3 Adenosin Rezeptor zu verabreichen.

Der EP 1 021 084 B1 ist ein Verfahren zur Behebung oder Reduzierung von Ischämieschäden an einem Organ zu entnehmen. Hierbei wird das geschädigte Organ mit einer gepufferten physiologischen Lösung gespült, um saure Produkte, die sich in dem Organ während der Sauerstoffarmut angesammelt haben, zu entfernen.

Der US 2005/0101907 A1 ist ein automatisches System zur Wiederbelebung eines Patienten zu entnehmen, bei dem die Zuführung einer einzigen Flüssigkeit in Bezug auf den Flüssigkeitszustrom in Abhängigkeit von am Patienten gewonnenen physiologischen Größen infundiert wird.

Ein vergleichbares, automatisches Infusionssystem zur Behandlung von Traumapatienten ist der US 5,938,636 zu entnehmen, bei der eine Infusionsflüssigkeit in Bezug auf Infusionsdruck und Infusionsfluss sensorerfasst und computerüberwacht dem Patienten verabreicht wird.

Der DE 10 2008 024 471 A1 ist eine Herz-Lungen-Bypasseinrichtung zu entnehmen, die mit einem Patienten über jeweils eine Röhre mit dem arteriellen sowie venösen Blutgefäßbereich verbindbar ist. Extrakorporal zwischen den Röhren ist ein Blutleitweg vorgesehen, längs dem eine bidirektional betreibbare Pumpe sowie ein Fluidreservoir vorgesehen sind. Zusätzlich sind steuerbare und mit Sensoren versehene Fluidsteuerventile längs des Blutleitweges angeordnet. Durch den bidirektionalen Pumpenbetrieb übernehmen die Lungen die Funktion des Oxygenators der Herz-Lungen-Bypasseinrichtung.

Die US 5,308,320 beschriebt ein tragbares Wiederbelebungsgerät bei Herzstillstand bei Patienten mit folgenden Mitteln: ein Blutentnahmemittel, eine Pumpe, um das Blut innerhalb des Gerätes zu bewegen, Mittel zur Sauerstoffanreicherung des Blutes sowie Mittel, um das Sauerstoff angereicherte Blut wieder in die Blutbahn zurückzuführen.

### Darstellung der Erfindung

Es besteht die Aufgabe eine Vorrichtung zu schaffen, mit der eine ganzheitliche Wiederbelebung eines Patienten ohne Risiko des Auftretens von ischämischen Schädigungen möglich wird und dies auch bei einem zeitlichen Abstand zwischen dem Eintritt des Herzstillstandes und dem Einleiten von wiederbelebenden Maßnahmen der deutlich größer ist als das bisher kritische Zeitfenster von 3 bis 5 Minuten. Die Vorrichtung soll die Wiederbelebung möglichst vollautomatisch ermöglichen, so dass vor Ort keine komplizierten therapeutischen Vorkehrungen zu treffen sind. Auch soll die Vorrichtung möglichst leichtgewichtig, portabel und autark bedienbar ausgebildet sein, um auf diese Weise auch als Instrument für die Notfallmedizin vor Ort dienen zu können. Ferner soll die Vorrichtung nicht nur bei einem Herzstillstand, sondern auch bei einer Herzminderleistung oder einem Schlaganfall unter Zugrundelegung der gleichen Wirkprinzipien einsetzbar sein.

Die der Erfindung zugrunde liegende Aufgabe wird durch eine Vorrichtung mit den Merkmalen des Anspruchs 1 gelöst. Den Erfindungsgedanken weiterbildende Merkmale sind Gegenstand der Unteransprüche sowie der weiteren Beschreibung, insbesondere unter Bezugnahme auf die Ausführungsbeispiele, zu entnehmen.

Der Erfindung liegt die Idee zugrunde anhand wenigstens einer sensorisch unterstützten Blutanalyse vom Blutes des Individuums eine individuell vom Blutanalyseergebnis abhängige Einflussnahme auf das vom Individuum entnommene Blut dahingehend auszuüben, dass dem vom Individuum entnommenen Blut eine individuell ausgewählte Substanz oder eine individuell angefertigte Substanzmischung zum Erhalt eines so genannten modifizierten Blutes beigegeben bzw. zugemischt wird. Das so gewonnene modifizierte Blut wird als Reperfusat in das Individuum reperfundiert mit dem Ziel, dass bei Aktivierung des natürlichen oder künstlich unterstützten Blut-Kreislaufsystems oder dass bei einem initialen Durchströmen von kurz- oder längerfristig vom natürlichen Blut-Kreislaufsystem abgekoppelten Gewebebereichen mit Blut, andernfalls auftretende Ischämiebedingte Gewebeschädigungen teilweise oder vollständig vermieden werden. Ausgehend von einer Vorrichtung zur Behandlung eines Individuums mit Herzstillstand oder mit Schlaganfall mit einem am Individuum applizierbaren Blutentnahmemittel, zur Entnahme zumindest eines Teils des Blutes aus dem Individuum, einer mittel- oder unmittelbar mit dem Blutentnahmemittel verbundenen Analyseeinheit zur Erfassung und Bereitstellung wenigstens einer Eigenschaft des Blutes in Form eines Blutanalyseergebnis, einer Wirkeinheit, die mittel- oder unmittelbar mit einem am Individuum applizierbaren Rückführungsmittel verbunden ist und zur Abgabe einer Substanz über das Rückführungsmittel an das Individuum ausgebildet ist, zeichnet sich die lösungsgemäße Vorrichtung dadurch aus, dass die Wirkeinheit wenigstens eine Reservoireinheit umfasst, die wenigstens zwei Substanzen bevorratet. Die Reservoireinheit ist mit einer Dosiereinheit kombiniert, die zumindest unter Berücksichtigung eines von der Analyseeinheit ermittelten Blutanalyseergebnisses aus den wenigstens zwei Substanzen wenigstens eine Substanz auswählt oder eine Mischung aus den wenigstens zwei der Substanzen anfertigt. Die wenigstens eine ausgewählte Substanz oder die Mischung ist anschließend mittel- oder unmittelbar über das Rückführungsmittel in das Individuum einbringbar.

Das Individuum ist insbesondere ein menschlicher oder tierischer Patient. Die Begriffe "Patient" und "Individuum" werden vorliegend synonym verwendet. Als Herzminderleistung wird beispielsweise eine traumatische oder krankheitsbedingte Minderleistung des Herzens verstanden, die beispielsweise durch einen Herzinfarkt, einen kardiogenen Schock oder eine Herzinsuffizienz hervorgerufen sein können.

In einer Variante weist die Vorrichtung ferner eine wenigstens eine Eigenschaft des Blutes des Individuums erfassende Sensoreinheit auf, die ein Sensorsignal generiert, das dann von der Analyseeinheit ausgewertet und als Blutanalyseergebnis bereitgestellt wird. Die Analyseeinheit ist grundsätzlich dazu geeignet, mit einer Sensoreinheit derart zusammenzuwirken, dass ein von der Sensoreinheit ermitteltes bzw. generiertes Sensorsignal von der Sensoreinheit auf die Analyseeinheit übertragbar ist. Grundsätzlich ist die Vorrichtung mit bereits bekannten Sensoren realisierbar.

Die Sensoreinheit kann eine Vielzahl von Sensoren umfassen, von denen jeder Sensor wenigstens einen Parameter bzw. eine Eigenschaft des Blutes erfasst.

In einer Variante ist die Sensoreinheit in Form einer nichtinvasiven, mittel- oder unmittelbar am Individuum anbringbaren Baueinheit ausgebildet.

Das zur Blutentnahme erforderliche Entnahmemittel ist dafür vorgesehen und eingerichtet dem Individuum zumindest einen Teil des Blutes zu entnehmen, bevor das Blut durch die Wirkeinheit beeinflusst wird. Dazu ist das Entnahmemittel beispielsweise invasiv am Individuum applizierbar ausgebildet. Dabei können mindestens zwei Liter, insbesondere mindestens drei Liter, insbesondere mindestens vier Liter, insbesondere das gesamte Blut, das sich in den Adern des Individuums befindet, entnommen werden.

Durch das ebenfalls vorgesehene Rückführungsmittel kann das entnommene und behandelte oder beeinflusste Blut, d.h. das modifizierte Blut wieder in den Körper des Individuums rückgeführt werden. Auch das Rückführungsmittel ist invasiv am Individuum applizierbar ausgebildet.

Zum einen ist es denkbar, die wenigstens eine Substanz in Form einer Zugabe an das im Patienten befindliche.Blut zuzugeben, beispielsweise im Wege einer Injektion oder Infusion, oder aber das im Patient befindliche Blut wird über ein Entnahmemittel aus dem Patient entnommen und extrakorporal mit der wenigstens einen Substanz angereichert bzw. behandelt und im Anschluss daran in Form modifizierten Blutes wieder in den Patient reperfundiert. Ebenso ist es denkbar das Blut im Wesentlichen vollständig aus dem Patient zu entnehmen und anstelle dessen das modifizierte Blut oder eine individuell auf den Patient abgestimmte, wenigstens eine Substanz enthaltene Lösung dem Patient direkt zuzuführen, um anschließend den Prozess der Reanimation ohne Gewebeschädigungen einleiten zu können.

In einer Ausgestaltung ist die Sensoreinheit mittel- oder unmittelbar mit dem Entnahmemittel verbunden.

Zu Zwecken einer individuell auf die Patientensituation abgestellte Modifikation bzw. Manipulation des patienteneigenen Blutes wird die Wirkeinheit in einer Variante von einer Auswerte- und Kontrolleinheit auf der Grundlage des Analysenergebnisses, das den aktuellen Patientenzustand repräsentiert, entsprechend angesteuert bzw. geregelt.

In vorteilhafter Weise sind die Analyseeinheit, die Wirkeinheit sowie die Auswerte- und Kontrolleinheit als portable und einheitliche Baueinheit ausgeführt, bei der vorzugsweise die Sensoreinheit Teil der einheitlichen Baueinheit ist.

Die Sensoreinheit generiert ein die wenigstens eine Eigenschaft des dem Patienten entnommenen Blutes repräsentierendes Sensorsignal, das leitungsgebunden oder drahtlos an die Analyseeinheit übermittelbar ist, wobei die Auswerte- und Kontrolleinheit auf Grundlage des Analyseergebnisses Steuer- oder Regelsignale generiert. Diese können beispielsweise dazu dienen, die Art und/oder Menge der zuzugebenden Substanz bzw. Substanzmischung auszuwählen bzw. zu dosieren. Hierbei erfasst die Sensoreinheit zumindest einen der folgenden Blutparameter: pH-Wert, Sauerstoffpartialdruck (pO₂), Kohlendioxidpartialdruck (pCO₂), Kaliumgehalt (K), Natriumgehalt (Na), Calciumgehalt (Ca), Basenüberschuss (BE), Lactatwert (La) und Glucosegehalt (Gu).

Die Vorrichtung ist vorzugsweise als portable und einfach zu bedienende, insbesondere vollständig autonom funktionierende Einheit ausgebildet, so dass sie nicht notwendigerweise ausschließlich von medizinischem Fachpersonal zu bedienen ist. Eine Ausführungsform besteht aus einer portabel handhabbaren Baueinheit, von der lediglich zwei Leitungen abgehen, die patientenseitig mit dem Blutgefäßsystem zu verbinden sind. Eine Leitung dient zur Entnahme des Blutes aus dem Patienten, über die das Blut des Patienten in die Baueinheit automatisch abfließt, in der eine Analyse des Blutes sowie eine entsprechende Modifikation des Blutes erfolgt. Über die andere Leitung wird das entsprechend modifizierte Blut in den Patienten wieder reperfundiert. Alternativ ermöglicht diese Vorrichtung aber auch, dass bevor das modifizierte Blut in den Patienten rückgeführt wird, dem Patienten eine individuell zusammengestellte Perfusionslösung verabreicht werden kann, deren Zusammenstellung von dem Analyseergebnis des patienteneigenen Blutes abhängt. So ist es für den Behandlungserfolg denkbar, in einem ersten Schritt das Blut weitgehend vollständig durch die individuell zusammengestellte Perfusionslösung zu substituieren. Erst im weiteren Verlauf der Behandlung wird dem Patienten dann das vorstehend erläuterte modifizierte Blut verabreicht, ggf. nach erfolgreichem Durchführen von weiteren Reanimationsmaßnahmen.

In einer Variante ist die Wirkeinheit dazu vorgesehen und eingerichtet, die wenigstens eine Substanz dosiert, temperiert und/oder druckbeaufschlagt in das Individuum oder in das Blut abzugeben.

Vorzugsweise ist ferner eine Monitoreinheit bzw. Überwachungseinheit vorgesehen, die mindestens ein Messmittel zur Erfassung wenigstens eines Parameters des Individuums aufweist, der aus der Gruppe der physiologischen Parameter des Individuums, umfassend arteriellen Mitteldruck, zentralvenösen Druck, Pulmonalarterien-Druck, Sauerstoffsättigung und Bluttemperatur, ausgewählt ist, wobei die Monitoreinheit mit einer Auswerte- und Kontrolleinheit zum Zwecke jeweils wenigstens eines einseitigen Informationsaustausches in Verbindung steht.

Grundsätzlich wird dem Patienten Blut als Körperflüssigkeit entnommen. Allerdings sind die Ausgestaltungen der Vorrichtung, die sich auf Blut als Körperflüssigkeit beziehen, auch auf andere Körperflüssigkeiten in analoger Weise übertragbar.

Das am Patienten zu applizierende Blutentnahmemittel ist vorzugsweise über einen Blutleitungsweg, im einfachsten Fall in Form einer sterilen Hohlleitung, mit dem am Patienten applizierbaren Blutrückführungsmittel verbunden, wobei insbesondere längs des Blutleitungsweges die Reservoireinheit der Wirkeinheit vorgesehen ist, die wenigstens zwei Substanzen bevorratet und aus der wenigstens eine auszuwählende Substanz bzw. eine Substanzmischung in den Blutleitungsweg bzw. in das Rückführmittel zugebbar ist.

Längs des Blutleitungsweges kann zudem eine Wärmetauschereinheit vorgesehen sein, die mittelbar oder unmittelbar mit dem Rückführungsmittel verbunden ist. Auch dient wenigstens ein längs des Blutleitungsweges integriertes Fördermittel für einen einstellbaren Blutfluss längs des Blutleitungsweges, um einen einfachen Transport des Blutes aus dem Körper des Individuums und des modifizierten Blutes in den Körper des Individuums zu gewährleisten. Bevorzugter Weise ist zur Blutrückführung in den Körper wenigstens ein weiteres, separates Fördermittel vorgesehen, mit dem eine individuelle und vor allem unabhängige Fördercharakteristik in Bezug auf Pulsabilität, Strömungsdruck und -geschwindigkeit einstellbar ist.

Zur Ansteuerung aller vorhandenen Fördermittel sowie der Wärmetauschereinheit generiert die Auswerte- und Kontrolleinheit weitere Steuersignale, so dass letztlich der Strömungsdruck, die Flussrate und/oder die Temperatur der in den Patienten zugeführten wenigstens einen Substanz bzw. des modifizierten Blutes vorgebbar einstellbar sind.

Auch die Analyseeinheit mit ihrer Sensoreinheit ist längs des Blutleitungsweges angeordnet, so dass das Blut in Hinblick auf einzelne Blutparameter, insbesondere in Hinblick auf eine Vielzahl von Blutparametern, analysiert und ein Blutanalyseergebnis zur weiteren Bewertung bereitgestellt werden kann. Die Analyse erfolgt vorzugsweise online, d.h. in situ, während das Blut mittels des Blutentnahmemittels dem Patienten entnommen wird.

Die mit der Reservoireinheit verbundene Dosiereinheit bringt zumindest unter Berücksichtigung des von der Analyseeinheit ermittelten Blutanalyseergebnisses eine vorbestimmbare Menge der wenigstens einen Substanz aus der Reservoireinheit in den Blutleitungsweg bzw. das Rückführungsmittel einbringt. Das heißt, mit Hilfe der Dosiereinheit wird ein von dem aktuellen Zustand des zu behandelnden Patienten individuell zusammengestelltes Reperfusat erzeugt, das dem Patienten dann über das applizierte Blutrückführungsmittel reperfundiert wird.

Insbesondere bei der Reperfusion von modifizierten Blut in den Patienten, aber auch bei der bloßen Verabreichung einer Perfusionsflüssigkeit, erfolgt sowohl die Beimischung von wenigstens einer, insbesondere einer Vielzahl von Substanzen zum patienteneigenen Blut, bzw. in die Perfusionlösung, als auch die Reperfusions selbst, hinsichtlich der Wahl des Reperfusionsdruckes, der Flussrate, der Reperfusionsdauer sowie der Temperatur des Reperfustes, unter Berücksichtigung und in Abgleich an das aktuell sensoriell erfasste Blutbild des Patienten. Dazu kann beispielsweise die bereits erläuterte Monitoreinheit dienen. Somit kann die Wahl und Einstellung der physiologischen Reperfusionsbedingungen auch unter Berücksichtigung der von der Monitoreinheit erfassten physiologischen Parameter vorgenommen werden.

Zur Bewertung des seitens der Analyseeinheit ermittelten Blutanalyseergebnisses und zur Bestimmung von Art und der jeweiligen Menge der dem patienteneigenen Blut bzw. der Perfusionslösung zuzugebenden Substanzen dient die bereits erwähnte Auswerte- und Kontrolleinheit, die beispielsweise sowohl mit der Analyseeinheit als auch mit der Dosiereinheit zum Zwecke jeweils wenigstens eines einseitigen Informationsaustausches in Verbindung steht. Innerhalb der Auswerte- und Kontrolleinheit wird das Blutanalyseergebnis unter bestimmten vorgegebenen Bewertungskriterien evaluiert, in denen auch die mittels der Monitoreinheit erfassten physiologischen Patientenparameter Berücksichtigung finden können.

Für den wenigstens einseitigen Informationsaustausch zwischen den einzelnen Vorrichtungskomponenten dienen entweder Datenübertragungsverbindungen in Form konventioneller Datenübertragungsleitungen oder drahtlose Übertragungstechniken.

Als Ergebnis der Evaluation des Blutanalyseergebnisses generiert die Auswerte- und Kontrolleinheit Steuersignale, die an die Dosiereinheit zum Zwecke der Auswahl von Art und Menge der in den Blutleitungsweg einzubringenden Substanzen, übertragen werden. Der Begriff der "Dosiereinheit" ist in diesem Zusammenhang als ein technisches Mittel zu verstehen, mit dem es möglich ist, aus einer Vielzahl von Substanzen, die in einer Reservoireinheit in jeweils getrennten Reservoirkammern bevorratet sind, unter Vorgabe eines definierten Mischungsplans, in dem eine Auswahl jeweiliger Substanzen sowie die Menge der jeweils zuzuführenden Substanz festgelegt ist, in eine Mischung überzuführen, die dem patienteneigenen Blut letztlich beizumischen ist. Nicht notwendigerweise ist es erforderlich, jeweils ausgewählte Substanzen vor der Zugabe zum patienteneigenen Blut vorzumischen, denkbar ist auch eine separate, dosierbare Zuführung jeweils einzeln ausgewählter Substanzen in den Blutleitungsweg.

Vorzugsweise sieht die Dosiereinheit wenigstens einen Mischbehälter vor, der über einzeln ansteuerbare Dosiermittel verfügt, die mit den einzelnen Reservoirkammern verbunden sind. Innerhalb des Mischbehälters erfolgt die Herstellung einer der Körperflüssigkeit des Individuums zuzugebenden Substanzmischung, die in Form einer Lösung, einer Suspension oder Emulsion vorliegen kann. So sind die in den Reservoirkammern jeweils bevorrateten Substanzen nicht notwendigerweise flüssig, vielmehr können einzelne Substanzen in fester Form bzw. in Pulverform sowie auch in gasförmiger Phase vorliegen. Zur Bevorratung in den einzelnen Reservoirkammern bieten sich beispielsweise die nachfolgenden Substanzen oder Substanzklassen an, aus denen eine individuelle Auswahl zur Herstellung einer der Körperflüssigkeit des Individuums beizumischenden Substanzmischung getroffen werden kann: alkalische oder saure Pufferlösungen, den Natrium-, Kalium- und/oder Kalziumgehalt der Körperflüssigkeit beeinflussende Substanzen, blutverdünnende Substanzen, freie Radikalfänger, Glutamat, Aspartat, Herzrhythmus-stabilisierende Substanzen wie beispielsweise Lidocain, die Leukozytenzahl beeinflussende Mittel, osmotisch aktive Substanzen in Form von Salzen, Glukose und/oder Proteinen.

In einer Variante ist insbesondere längs eines Blutleitungsweges zwischen dem Blutentnahmemittel und dem Rückführungsmittel eine Filtereinheit zur Blutfilterung vorgesehen. Diese Filtereinheit kann beispielsweise einen Leukozytenfilter aufweisen.

In einer Variante ist längs eines Blutleitungsweges zwischen dem Blutentnahmemittel und dem Rückführungsmittel eine Bypassleitung vorgesehen, die dem Rückführungsmittel unmittelbar vorgeordnet ist. Durch die Bypassleitung kann ein Teil des in das Individuum rückzuführenden und modifizierten Blutes in die Analyseeinheit zugeführt werden. Im Falle eines über eine vorgebbare Toleranzschwelle abweichenden Soll-/Istwert-Vergleiches für wenigstens einen erfassbaren Blutparameter kann die Dosiereinheit dann eine Änderung an der Mengenzugabe der wenigstens einen Substanz in den Blutleitungsweg vornehmen.

In einer Variante sind die einzelnen Komponenten der Vorrichtung derart kompakt und leichtgewichtig ausgebildet, dass die Vorrichtung portabel ist.

In einer Variante ist längs eines Blutleitungsweges zwischen dem Blutentnahmemittel und dem Rückführungsmittel eine Sauerstoffanreicherungs- und - abreicherungseinheit für das Blut angeordnet, um den Sauerstoffgehalt des rückzuführenden modifizierten Bluts den jeweiligen Bedürfnissen entsprechend anpassen zu können.

Eine Ausführungsform der lösungsgemäßen Vorrichtung sieht ein portables Grundmodul vor, das im Weiteren als CIRD (als Akronym für die englische Übersetzung "Controlled Integrated Resuscitation Device" des Begriffs "Kontrollierte, integriert-gesteuerte Reanimationsvorrichtung") bezeichnet wird. Das CIRD-Grundmodul weist einen extrakorporalen Blutleitungsweg auf, der über geeignete Blutentnahme- und (Blut)Rückführungsmittel an den patienteneigenen Blutkreislauf, insbesondere im Bereich der Femoralarterie sowie Femoralvene, applizierbar ist. Das CIRD-Grundmodul sieht längs des extrakorporalen Blutleitungsweges ein Fördermittel zur Aufrechterhaltung eines Blutflusses, einen Oxygenator zur Blutsauerstoffanreicherung sowie eine Einheit zur CO₂-Abreicherung und schließlich einen Leukozytenfilter vor, und kann in modularer Erweiterung mit der vorstehend erläuterten Blutanalyseeinheit sowie den vorstehend beschriebenen Reservoir- und Dosiereinheiten ergänzt werden.

Mit Hilfe einer derartigen Vorrichtung, zu deren automatischen Betrieb und Kontrolle die gleichfalls vorstehend erwähnte Auswerte- und Kontrolleinheit zumeist in Ausbildung einer Rechnereinheit vorgesehen ist, ist es möglich, insbesondere das aus einem Patienten mit Herzstillstand ausgeleitete Blut schnell und automatisch zu analysieren und festzustellen, welche exakte Zusammensetzung von zusätzlichen Substanzen dem patienteneigenen Blut beizumischen ist. Aus dem Ergebnis der Blutanalyse, sowie ggf. unter Berücksichtigung der sensorisch erfassten physiologischen Patientenparameter wird schließlich ein individuell auf den Patienten abgestimmtes Reperfusat automatisch hergestellt und dem Patienten unter optimierten Bedingungen in Bezug auf Pulsabilität, Strömungsdruck, Flussrate und/oder Temperatur zugeführt. Hierdurch werden ideale Bedingungen für die initiale Reperfusion in Hinblick auf den Reperfusionsdruck, den Reperfusionsfluss, die Reperfusionstemperatur sowie die Reperfusionsdauer geschaffen.

Da die lösungsgemäße Vorrichtung eine kontinuierliche Überwachung des patienteneigenen Blutes ermöglicht, können in situ bzw. online, das heißt kontinuierlich, Anpassungen in Bezug auf die Bedingungen der Reperfusion sowie der Zusammensetzung des Reperfusates an den aktuellen Zustand des reperfundierenden Patienten, vorgenommen werden. Insbesondere durch die In-situ- bzw. Online-Messung bestimmter Blutwerte, wie beispielsweise der Konzentration an Kaliumionen, Lactat, Glucose oder des pH-Werts etc. sowie durch die kombinierte Messung hämodynamischer Parameter mit Hilfe geeigneter Monitorsensoren, die den Strömungswiderstand, die Temperatur, den Strömungsfluss sowie den Strömungsdruck etc. innerhalb des Blutleitungsweges erfassen, können automatische Anpassungen im Rahmen der kontrollierten Ganzkörper-Reperfusion vorgenommen werden.

Im Rahmen von Experimenten an Schweinen ist die lösungsgemäße Vorrichtung zur extrakorporalen kontrollierten Ganzkörper-Reperfusion mit großem Erfolg erprobt worden. So konnten Tiere 15 Minuten nach einem kontrolliert herbeigeführten Herzstillstand unter normothermen Bedingungen erfolgreich wiederbelebt werden, ohne erkennbare oder messbare Organschäden oder neurologische Schäden. Diese Experimente zeigten erstmals, dass es möglich ist, auch 15 Minuten nach dem Eintritt des Herzstillstandes eine vollständige funktionale neurologische Wiederherstellung zu erlangen, eine Tatsache, die im scharfen Kontrast zum Paradigma der heute üblichen konventionellen Behandlungsmethoden und der Limitationen steht. Insbesondere durch die leichtgewichtig und portable Ausbildung der lösungsgemäßen Vorrichtung eröffnen sich für die Notfallmedizin völlig neue Perspektiven, die dazu führen können, dass sehr viele Patienten, die aus heutiger Sicht keine Chance auf Wiederbelebung und/oder vollständige Genesung haben, zukünftig nicht nur gerettet, sondern auch ohne neurologische Schäden wieder hergestellt werden können.

Beschrieben wird hier beispielsweise auch ein Verfahren zur Behandlung eines Individuums mit Herzminderleistung Herzstillstand oder Schlaganfall, bei dem dem Individuum Blut entnommen wird, das einer Blutanalyse unterzogen wird, wobei zumindest unter Berücksichtigung eines von der Analyseeinheit ermittelten Blutanalyseergebnisses und wenigstens eines Bewertungskriteriums aus wenigstens zwei bevorrateten Substanzen Art und Menge wenigstens einer Substanz oder Substanzmischung bestimmt werden, die entweder in dosierter Form dem entnommenen Blut zum Erhalt eines modifizierten Blutes beigegeben wird, das in das Individuum reperfunidert wird oder die in Form einer Perfusionslösung anstelle des entnommenen Blutes in das Individuum reperfundiert wird.

In einem Beispiel des Verfahrens wird dem Individuum das Blut vor der Erfassung der Eigenschaft entnommen und nach der Abgabe der wenigstens einen ausgewählten Substanz in das Blut zurückgeführt.

In einem Beispiel des Verfahrens erfolgt die Erfassung, Bewertung und Beeinflussung in einem geschlossenen Regelkreislauf.

In einem Beispiel des Verfahrens wird das Analyseergebnis mit einem Sollwert verglichen und bei einer quantitativ vorgebbaren Abweichung vom Sollwert eine die dem Analyseergebnis zugrundeliegende Eigenschaft des Blutes beeinflussende Substanz ausgewählt und mit einer von der quantitativen Abweichung abhängigen Menge in das Individuum oder in das Blut abgegeben.

In einem Beispiel des Verfahrens werden dem Individuum mindestens 2 Liter, insbesondere 3 Liter, insbesondere 4 Liter, insbesondere im Wesentlichen sämtliches Blut, das sich in den Adern des Individuums befindet, entnommen, bevor eine Reperfusion des modifizierten Blutes erfolgt.

In einem Beispiel des Verfahrens wird dem Patienten das Blut über einen Zeitraum von 10 Sekunden bis 3 Minuten, insbesondere von 20 Sekunden bis 2 Minuten, insbesondere von 30 Sekunden bis 1 Minute entnommen.

In einem Beispiel des Verfahrens wird von dem modifizierten Blut ein Anteil zu Zwecken einer wiederholten Blutanalyse abgezweigt, und bei Feststellung einer Abweichung zwischen wenigstens einem erfassten Blutparameter als Eigenschaft des Blutes von einem Sollwert eine Korrektur von Art und/oder Menge der wenigstens einen zugegebenen Substanz vorgenommen wird.

In einem Beispiel des Verfahrens erfolgen die Vorgänge der Blutentnahme, der Blutanalyse, der Zugabe der wenigstens einen Substanz zu dem Blut oder der Perfusionslösung sowie das Reperfundieren des modifizierten Blutes oder der Perfusionslösung in situ.

In einem Beispiel des Verfahrens wird zusätzlich zur Analyse der Eigenschaft des Blutes wenigstens ein physiologischer Parameter des Individuums erfasst, wobei die Art und Menge der wenigstens einen abzugebenden Substanz und/oder der Druck, die Flussrate und/oder die Temperatur des in das Individuum zu reperfundierenden modifizierten Blutes unter Berücksichtigung des Analyseergebnisses der Eigenschaft des Blutes und dem physiologischen Parameter des Individuums gewählt werden.

In einem Beispiel des Verfahrens wird die Reperfusion des modifizierten Blutes oder der Perfusionslösung in das Individuum an wenigstens zwei unterschiedlichen Körperbereichen mit jeweils unterschiedlichen Reperfusionsparametern in Bezug auf Druck, Flussrate, Temperatur und/oder Reperfusionsdauer durchgeführt.

In einem Beispiel des Verfahrens erfolgt die Blutentnahme mit einer kontrollierten Flussrate von wenigstens 1 l/min, insbesondere von 6 bis 8 l/min.

Sämtliche der vorgenannten Beispiele sind miteinander in beliebiger Weise und Reihenfolge kombinierbar.

### Kurze Beschreibung der Figuren

Die Erfindung wird nachstehend ohne Beschränkung des allgemeinen Erfindungsgedankens anhand von Ausführungsbeispielen unter Bezugnahme auf die Zeichnungen beschrieben. Es zeigen:
- Fig. 1: ein Blockschaltbild einer Illustration einzelner Komponenten eines Ausführungsbeispiels,
- Fig. 2: eine schematische Darstellung der Analyseeinheit,
- Fig. 3: eine schematische Darstellung einer Reservoir- und Dosiereinheit und
- Fig. 4: ein verallgemeinertes Blockschaltbild eines Ausführungsbeispiels.

### Wege zur Ausführung der Erfindung, gewerbliche Verwendbarkeit

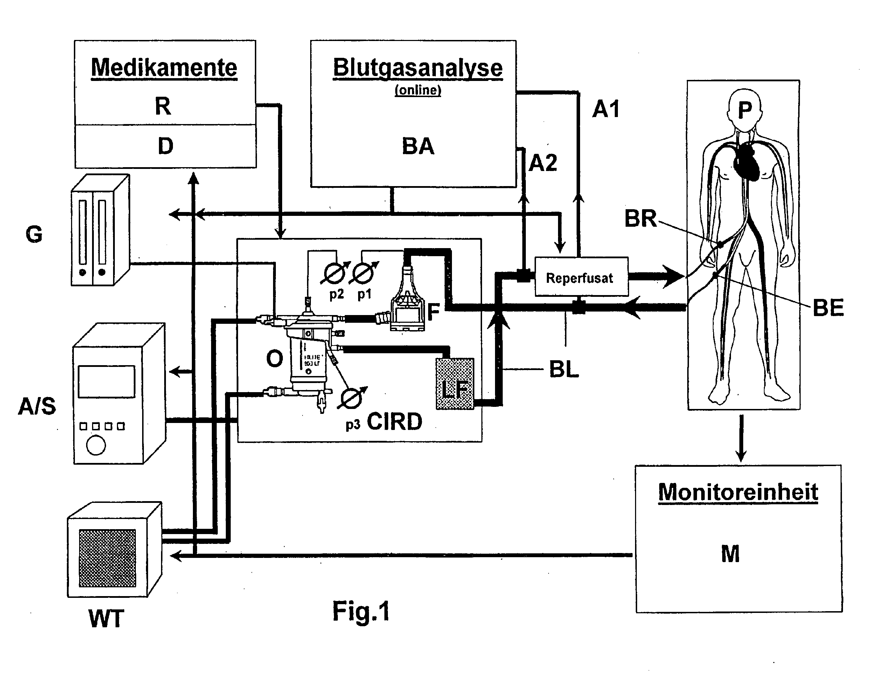

In Figur 1 ist eine Blockbilddarstellung zur Illustration aller Komponenten einer Ausführungsform einer lösungsgemäßen Vorrichtung dargestellt. Am Beispiel eines menschlichen Patienten P als Individuum, das einen Herzstillstand erlitten hat, soll die Anwendung der Vorrichtung näher erläutert werden. Im Bereich der Femoralvene wird am Patienten P zur Blutentnahme ein Blutentnahmemittel BE appliziert, von dem sich extrakorporal ein Blutleitungsweg BL erstreckt, der verschiedene technische Komponenten durchläuft und von dem an verschiedenen Stellen entsprechende Leitungsabzweige abgehen sowie in den an verschiedenen Stellen des Blutleitungsweges entsprechende Zuleitungen einmünden, auf die im weiteren näher eingegangen wird. Letztlich mündet der Blutleitungsweg BL wieder im Patienten, insbesondere im Bereich der Femoralarterie, an bzw. in die ein Blutrückführungsmittel BR als Rückführungsmittel appliziert ist.

Zur kontrollierten Blutentnahme aus dem Patienten P sowie zur Einstellung der Reperfusionbedingungen, unter denen die zu erläuternde Vorrichtung modifiziertes Blut bzw. Reperfusat in den Blutkreislauf des Patienten (wieder) zurückfördert, ist ein Fördermittel F längs des Blutleitungsweges BL vorgesehen, das insbesondere in Form einer Zentrifugalpumpe ausgebildet ist und als Bestandteil des leichtgewichtig und portabel ausgebildeten CIRD anzusehen ist. Das Fördermittel F ist über eine im Weiteren noch genauer zu beschreibenden Auswerte- und Kontrolleinheit A/K hinsichtlich der Förderleistung, Fördercharakteristik und Förderdauer, d.h. Druck, Fluss, Dauer sowie Pulsatilität variabel einstellbar. Ferner umfasst das portable CIRD einen Oxygenator O, mit dem das aus dem Patienten entnommene Blut mit Sauerstoff angereichert wird. Gleichfalls ist es möglich, mit Hilfe des Oxygenators in bestimmten Fällen das patienteneigene Blut auch von Sauerstoff zu entreichem. Ferner ist ein Gasblender G vorgesehen, der eine Beeinflussung des Blut-CO₂-Gehaltes, üblicherweise in Form einer Entreicherung des CO₂-Gehaltes im patienteneigenen Blut, vornimmt. Der Oxygenator O ist darüber hinaus für eine individuelle Temperierung der innerhalb des Blutleitungsweges BL geführten Blutströmung mit einer Wärmetauschereinheit WT verbunden, deren Wärmetauschercharakteristik durch die bereits erwähnte Auswerte- und Kontrolleinheit A/K kontrolliert beeinflussbar ist. Schließlich umfasst die portable Einheit CIRD einen Leukozytenfilter, durch den der Leukozytengehalt des patienteneigenen Blutes beeinflusst werden kann.

In dem vom Patienten P unmittelbar abführenden Blutleitungsweg BL ist eine erste Bypassleitung A1 vorgesehen, über die ein Teil des patienteneigenen Blutes in eine Analyseeinheit BA abgezweigt wird, in der das patienteneigene Blut sensoriell nach Maßgabe verschiedener Blutparameter untersucht wird.

In einer erweiterten Ausführungsform ist es denkbar die in Figur 1 erläuterte Vorrichtung durch weitere Funktionseinheiten zu ergänzen, die eine Modifikation bzw. Manipulation des patienteneigenen Blutes in der nachfolgenden Weise vorzunehmen in der Lage sind.

So werden Mittel vorgesehen zur mechanischen Beeinflussung des patienteneigenen Blutes durch extrakorporale Druckausübung auf den Patienten derart, dass die Druckausübung zeitlich und räumlich auf den Patienten gleichmäßig oder zeitlich variabel und räumlich selektiv vorgebbar erfolgt. Derartige Mittel zur mechanischen Beeinflussung des patienteneigenen Blutes können alternativ auch invasiv applizierbar und zur intrakorporalen Druckausübung auf das patienteneigene Blut ausgebildet sein.

Ferner können Mittel zur thermischen Beeinflussung des patienteneigenen Blutes zur extrakorporalen Temperierung des Patienten derart vorgesehen und ausgebildet werden, dass die Temperierung zeitlich und räumlich auf den Patienten gleichmäßig oder zeitlich variabel und räumlich selektiv vorgebbar erfolgt. Alternativ können diese Mittel zur thermischen Beeinflussung des patienteneigenen Blutes invasiv applizierbar ausgebildet sein zur intrakorporalen Temperierung des patienteneigenen Blutes, so dass die Temperierung zeitlich und räumlich innerhalb des Patienten gleichmäßig oder zeitlich variabel und räumlich selektiv vorgebbar erfolgt.

Bevorzugter Weise kann zur Blutrückführung in den Körper des Patieneten P längs des Blutleitweges vor oder nach dem Leukozytenfilter LF wenigstens ein weiteres, separates Fördermittel (nicht dargestellt) vorgesehen sein, mit dem eine individuelle und vor allem unabhängige Fördercharakteristik in Bezug auf Pulsabilität, Strömungsdruck und -geschwindigkeit einstellbar ist.

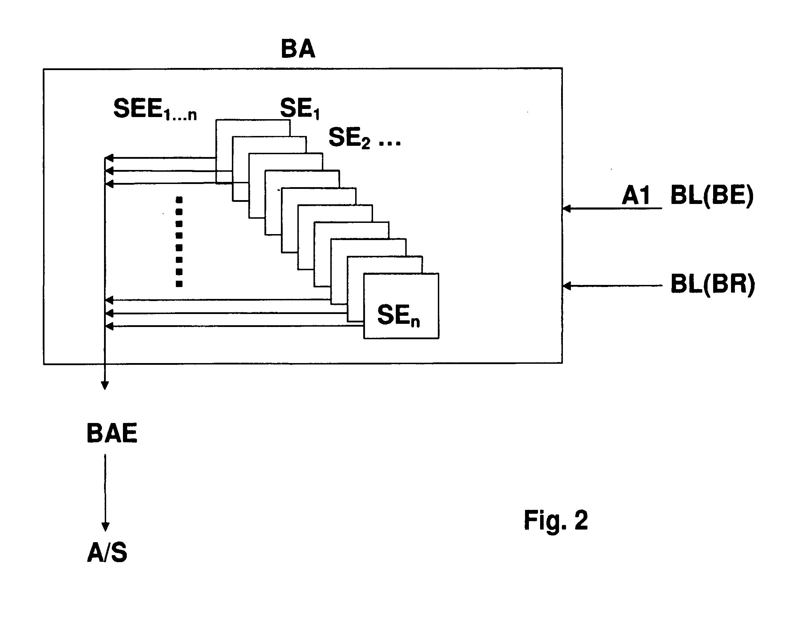

In Figur 2 ist zur näheren Erläuterung eine diesbezügliche Analyseeinheit BA schematisiert dargestellt. Es sei angenommen, dass über die Zuleitung A1 ein Teil des patienteneigenen Blutes in die das Blut analysierende Analyseeinheit BA gelangt. Innerhalb der Analyseeinheit BA ist, insbesondere in Form einer Sensoreinheit, eine Vielzahl einzelner Sensoren SE₁ bis SEₙ vorgesehen, die das Blut bezüglich n unterschiedlicher Blutparameter vermessen. In der Analyse- bzw. Sensoreinheit werden in vorteilhafter Weise an sich bekannte Sensoren zusammengefasst, von denen jeder einzelne wenigstens einen der nachfolgend in nicht abschließender Weise angeführten Blutparameter zu erfassen vermag: pH-Wert, Sauerstoffpartialdruck (pO₂), Kohlendioxidpartialdruck (pCO₂), Kaliumgehalt (K), Natriumgehalt (NA), Kalziumgehalt (Ca), die als BE bezeichnete Basenabweichung, auch als Basenüberschuss bzw. Basendefizit bezeichnet, anhand der die Erkennung stoffwechselbedingter Störungen des Säure-Basen-Haushaltes möglich ist, Lactatwert (La), Glucosegehalt (Gu), um nur einige zu nennen.

Jeder einzelne Sensor SE_{1...n} ermittelt einen das patienteneigene Blut charakterisierenden Blutparameter SEE_{1...n}, die zusammengefasst das sogenannte Blutanalyseergebnis BAE ergeben, das die aktuelle Qualität des patienteneigenen Blutes widerspiegelt. Das Blutanalyseergebnis BAE wird insbesondere über eine entsprechende Datenübertragungsleitung zur Auswerte- und Kontrolleinheit A/K übermittelt, in der das Blutanalyseergebnis BAE einer gesonderten Auswertung und Bewertung unterzogen wird, der medizinische Bewertungskriterien zugrunde gelegt werden.

Zweck der Vorrichtung ist es letztlich, das patienteneigene Blut durch Zugabe bestimmter Substanzen in einen modifizierten Zustand überzuführen, der dadurch charakterisierbar ist, dass das speziell modifizierte Blut bzw. das Reperfusat bei der initialen Reperfusion in den Patienten zu Zwecken seiner Wiederbelebung keinerlei Gewebeschädigungen hervorrufen soll. Überdies gilt es, möglicherweise bereits aufgetretene Ischämieschäden an bestimmten Gewebebereichen rückzubilden bzw. zu heilen.

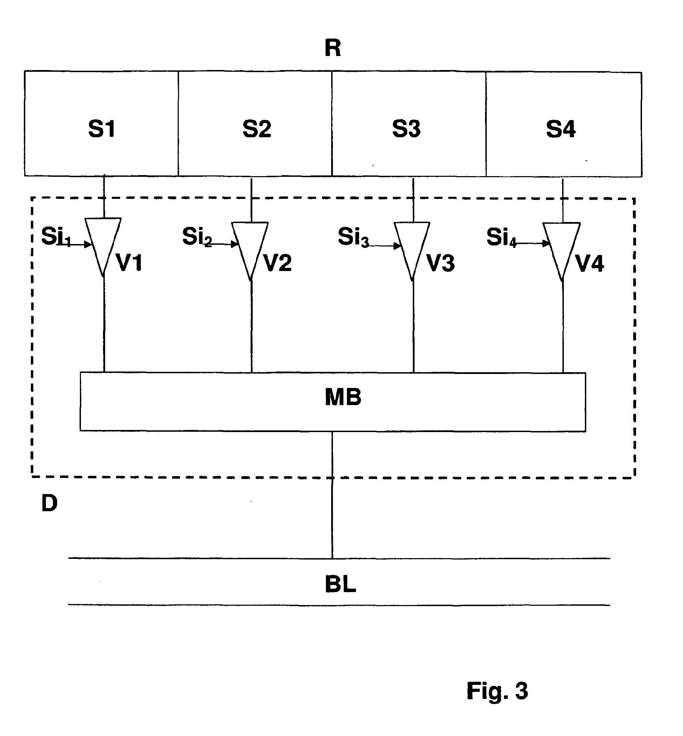

Innerhalb der Auswerte- und Kontrolleinheit A/K erfolgt ein Abgleich zwischen den aktuell sensoriell erfassten einzelnen Blutparametern SEE_{1...n} des patienteneigenen Blutes jeweils mit blutparameterspezifischen Referenz- bzw. Sollwerten, die es im Rahmen einer Modifikation des patienteneigenen Blutes herzustellen gilt. Unter Maßgabe einer derartigen Auswertung werden die Art und die Menge von den jeweils dem patienteneigenen Blut beizugebenden Substanzen ermittelt. Die Auswerte-/Kontrolleinheit steht in informeller Kommunikation mit einer Reservoireinheit R sowie einer damit kombinierten Dosiereinheit D, die beide in schematisierter Darstellungsform in Figur 3 gezeigt sind. Die Reservoireinheit R umfasst gemäß Figur 3 vier einzelne Reservoirkammem, in denen vier verschiedene Substanzen S₁, S₂, S₃ und S₄ bevorratet sind. Selbstverständlich können auch mehr derartige Reservoirkammern vorgesehen sein, d.h. allgemein Reservoirkammern zur Bevorratung von n unterschiedlichen Substanzen. Die einzelnen Reservoirkammern sind einzeln über Verbindungsleitungen mit einem Mischbehälter MB verbunden, wobei längs der Verbindungsleitungen Dosiermittel in Form von Sperrventilen V₁, V₂, V₃ und V₄ eingebracht sind. Unter Maßgabe des aktuellen Blutanalyseergebnisses BAE und des im Rahmen der Auswerte-/Kontrolleinheit ermittelten Zugabeerfordernisses für die dem patienteneigenen Blut beizumischenden Substanzen werden seitens der Auswerte-/Kontrolleinheit Steuersignale Si₁, Si₂, Si₃, Si₄ generiert, die der Betätigung der Dosiermittel V₁ bis V₄ dienen. Schließlich wird die im Mischbehälter MB bereitgestellte Mischung aus den einzelnen Substanzen in die Blutleitung BL eingebracht.

In einer Variante ist eine Monitoreinheit M, siehe Fig. 1, vorgesehen, die über am Patienten P geeignet angebrachte Sensoren physiologische Patientendaten erfasst, so beispielsweise den arteriellen Mitteldruck, den zentralvenösen Druck, den Pulmonalarterien-Druck, die Blutsauerstoffsättigung sowie die Körpertemperatur, um nur einige zu nennen. Auch die physiologischen Patientenparameter werden seitens der Monitoreinheit M zur weiteren Berücksichtigung zur Auswerte- und Kontrolleinheit A/K übertragen, unter deren Berücksichtigung die Auswerte- und Kontrolleinheit, die für die Dosiereinheit D bestimmten Steuersignale Si_{1...n} generiert.

Bevor das Reperfusat den Patienten P über den Blutleitungsweg BL wieder zugeführt wird, erfolgt mit Hilfe der Analyseeinheit BA eine Analyse des modifizierten Blutes bzw. Reperfusates, um sicherzustellen, dass den Patienten ein korrekt zusammengestelltes bzw. modifiziertes Reperfusat zugeführt wird. Hierzu ist unmittelbar stromauf zum Blutrückführungsmittel BR eine zweite Bypassleitung A2 vorgesehen, die einen Teil des modifizierten Blutes bzw. Reperfusates in die Analyseeinheit BA ableitet. Im Rahmen der Analyseeinheit BA erfolgt eine wiederholte sensorielle Erfassung der einzelnen Blutparameter SEE_{1...n}, die im Rahmen der Auswerte-/Kontrolleinheit A/K einer Soll-/Istwertanalyse unterzogen werden. Sollten Abweichungen auftreten erfolgt eine Korrektur der generierten Steuersignale Si₁, Si₂, Si₃, Si₄ zur Beeinflussung der Dosiermittel V₁ bis V₄.

Die Auswerte- und Kontrolleinheit generiert überdies auf der Grundlage des Blutanalyseergebnisses BAE sowie der seitens der Monitoreinheit M erfassten physiologischen Patientenparameter Steuersignale zur Ansteuerung sowohl des die Strömungscharakteristik innerhalb des Blutleitungsweges BL bestimmenden Fördermittels F, als auch des das Temperaturniveau des in den Patienten infundierenden Reperfusates bestimmenden Wärmetauschers WT, letztlich mit dem Ziel einer gewebeschonenden Reperfusion des modifizierten Blutes zurück in den Blutkreislauf des Patienten. Hierbei werden insbesondere der Strömungsdruck, der Strömungsfluss, die Pulsatilität, die Strömungsdauer und Temperatur des Reperfusates individuell auf den Patienten angepasst dimensioniert.

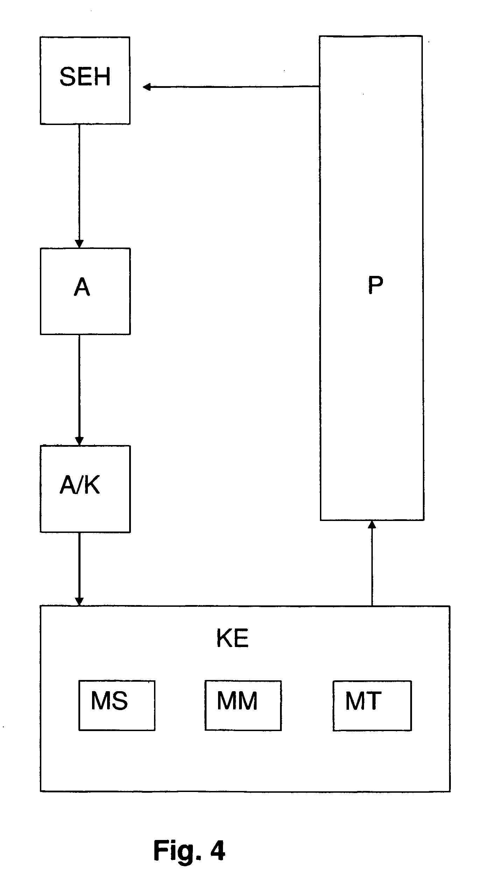

Figur 4 illustriert in einem schematischen Blockschaltbild eine weitere Ausführungsform der Vorrichtung. Mit Hilfe einer Sensoreinheit SEH werden Informationen von der Körperflüssigkeit, insbesondere Blut, von einem Patienten P entnommen. Die mit der Sensoreinheit SEH generierten Sensorsignale gelangen an eine Analyseeinheit A, die ein Analyseergebnis generiert, das den aktuellen Zustand der körpereigenen Flüssigkeit bzw. des Bluts repräsentiert. Das Analyseergebnis wird von einer Auswerte- und Kontrolleinheit A/K, beispielsweise unter Zugrundelegung wenigstens eines Bewertungskriteriums, beurteilt. Dann generiert die Auswerte- und Kontrolleinheit Steuer- bzw. Regelsignale zur kontrollierten Aktivierung einer die Körperflüssigkeit beeinflussenden Wirkeinheit KE, die sich insbesondere aus wenigstens einer der nachfolgenden Untereinheiten zusammensetzen kann: Ein Mittel zur Abgabe wenigstens einer Substanz an die Körperflüssigkeit MS, einem Mittel zur mechanischen Beeinflussung der Körperflüssigkeit MM und einem Mittel zur thermischen Beeinflussung der Körperflüssigkeit. Einzelne dieser Mittel können auch in einem gemeinsamen Mittel kombiniert sein. In Abhängigkeit der seitens der Auswerte- und Kontrolleinheit A/K aktivierbaren Mittel erfährt die körpereigene Flüssigkeit des Patienten P eine therapeutische Manipulation bzw. Modifikation zum Zwecke der Vermeidung ischämischer Gewebeschädigungen.

Die lösungsgemäße Vorrichtung ist insbesondere kompakt in möglichst einem einzigen Gehäuse ausgebildet, um eine möglichst einfache und vollautomatische Bedienung zu gewährleisten: Die Vorgänge der Blutentnahme, der Blutanalyse, der Zugabe der wenigstens einen Substanz zum patienteneigenen Blutes zum Erhalt eines modifizierten Blutes sowie das Reperfundieren des modifizierten Blutes erfolgt automatisch und in situ, ohne dass dabei weitere Kenntnisse über den wiederzubelebenden Patienten vorliegen müssen. Sämtliche Informationen für eine erfolgreiche Reperfusion erhält die Vorrichtung aus den erläuterten Sensordaten, die sich aus dem automatischen Blutscreening sowie den vom Patienten sensoriell abgreifbaren physiologischen Daten ergeben.

Mit Hilfe der lösungsgemäßen Vorrichtung lässt sich eine kontrollierte Ganzkörperperfusion durchführen, mit der sich die Ischämiedauer bis zur irreversiblen Schädigung einzelner Organe und sogar des gesamten Körpers deutlich gegenüber den derzeit engen Zeitgrenzen verlängern lässt.

### Bezugszeichenliste

- BE: Blutentnahmemittel
- BR: Blutrückführungsmittel
- BL: Blutleitungsweg
- BA: Analyseeinheit
- R: Reservoireinheit
- D: Dosiereinheit
- A1: Ableitung
- A2: Bypassleitung
- CIRD: Grundmodul der kontrollierten integriert-gesteuerten Reanimationsvorrichtung
- F: Fördermittel
- O: Oxygenator
- G: Gasblender
- LF: Leukozytenfilter
- A/K: Auswerte-/Kontrolleinheit
- WT: Wärmetauscher
- M: Monitoreinheit
- S1, S2...: Substanz
- Si_{1...}: Steuersignal
- V1, V2...: Dosiermittel, Ventil
- MB: Mischbehälter
- SE₁, SE₂...: Sensoren
- SEE₁,SEE₂...: Sensorergebnis
- BAE: Blutanalyseergebnis
- A: Analyseeinheit
- SEH: Sensoreinheit
- KE: Wirkeinheit
- MS: Mittel zur Abgabe wenigstens einer Substanz an die Körperflüssigkeit
- MM: Mittel zur mechanischen Beeinflussung der Körperflüssigkeit
- MT: Mittel zur thermischen Beeinflussung der Körperflüssigkeit

## Patentansprüche

1. Vorrichtung zur Behandlung eines Individuums mit Herz- oder Kreislaufstillstand oder mit Schlaganfall mit einem am Individuum (P) applizierbaren Blutentnahmemittel (BE), zur Entnahme zumindest eines Teils des Blutes aus dem Individuum, einer mittel- oder unmittelbar mit dem Blutentnahmemittel verbundenen Analyseeinheit (BA) zur Erfassung und Bereitstellung wenigstens einer Eigenschaft des Blutes in Form eines Blutanalyseergebnis (BAE), einer Wirkeinheit (KE), die mittel- oder unmittelbar mit einem am Individuum (P) applizierbaren Rückführungsmittel (BR) verbunden ist und zur Abgabe einer Substanz über das Rückführungsmittel (BR) an das Individuum ausgebildet ist,
**dadurch gekennzeichnet, dass** die Wirkeinheit (KE) wenigstens eine Reservoireinheit (R) umfasst, die wenigstens zwei Substanzen (S₁, S₂,...) bevorratet, dass eine mit der Reservoireinheit (R) kombinierte Dosiereinheit (D) vorgesehen ist, die zumindest unter Berücksichtigung eines von der Analyseeinheit (BA) ermittelten Blutanalyseergebnisses (BAE) aus den wenigstens zwei Substanzen (S₁, S₂,...) wenigstens eine Substanz auswählt oder eine Mischung aus den wenigstens zwei der Substanzen (S₁, S₂,...) anfertigt, wobei eine Auswerte- und Kontrolleinheit (A/K) vorgesehen ist, die zumindest mit der Analyseeinheit (BA) sowie der Dosiereinheit (D) draht- oder drahtlos in Informationsaustausch steht und zumindest auf Grundlage des Blutanalyseergebnisses (BAE) Steuersignale (Si₁, Si₂, ...) zur Ansteuerung der Dosiereinheit (D) derart generiert, dass durch die wenigstens eine ausgewählte Substanz oder die Mischung nach mittel- oder unmittelbaren Einbringen über das Rückführungsmittel (BR) in das Individuum bei einem initialen Durchströmen von kurz- oder längerfristig vom natürlichen Blut-Kreislaufsystem abgekoppelten Gewebebereichen mit Blut, andernfalls auftretende Ischämiebedingte Gewebeschädigungen teilweise oder vollständig vermeidbar sind.

2. Vorrichtung nach Anspruch 1,
**dadurch gekennzeichnet, dass** das am Individuum (P) applizierbare Blutentnahmemittel (BE) über einen Blutleitungsweg (BL) mit dem am Individuum (P) applizierbaren Rückführungsmittel (BR) verbunden ist, dass längs des Blutleitungsweges (BL) die Analyseeinheit (BA) sowie die Reservoireinheit (R) und Dosiereinheit (D) vorgesehen sind, und dass die wenigstens eine ausgewählte Substanz oder die Mischung vor Abgabe an das Individuum über das Rückführungsmittel (BR) mit dem aus dem Individuum entnommenen Blut zum Erhalt eines modifizierten Blutes vermischbar ist.

3. Vorrichtung nach Anspruch 2,
**dadurch gekennzeichnet, dass** längs des Blutleitungsweges (BL) wenigstens ein Fördermittel (F) für einen einstellbaren Fluss vorgesehen ist

4. Vorrichtung nach einem der Ansprüche 1 bis 3,
**dadurch gekennzeichnet, dass** die Analyseeinheit (BA) eine Vielzahl von Sensoren (SE₁, ... SEₙ) umfasst, von denen jeder wenigstens einen Blutparameter erfasst.

5. Vorrichtung nach Anspruch 4,
**dadurch gekennzeichnet, dass** die Analyseeinheit (BA) zumindest einen der nachfolgenden Blutparameter sensoriell erfasst: pH-Wert, Sauerstoffpartialdruck (pO₂), Kohlendioxidpartialdruck (pCO₂), Kaliumgehalt (K), Natriumgehalt (Na), Calziumgehalt (Ca), base excess (BE), Lactatwert (La), Glucosegehalt (Gu).

6. Vorrichtung nach einem der Ansprüche 1 bis 5,
**dadurch gekennzeichnet, dass** die Reservoireinheit (R) eine Vielzahl von Substanzen (S₁, S₂,...) bevorratet, die in fester, flüssiger oder gasförmiger Form vorliegen und in getrennten Reservoirkammern bevorratet sind, die vereinzelt oder in Kombination mittels der Dosiereinheit (D) dosiert zugebbar sind.

7. Vorrichtung nach Anspruch 6,
**dadurch gekennzeichnet, dass** die Dosiereinheit (D) wenigstens einen Mischbehälter (MB) vorsieht, der über einzeln steuerbare Dosiermittel (V₁, V₂, ...) mit den einzelnen Reservoirkammern verbunden ist, und dass der wenigstens eine Mischbehälter (MB) über ein weiteres steuerbares Dosiermittel mittel- oder unmittelbar mit dem Rückführungsmittel (BR) verbunden ist.

8. Vorrichtung nach einem der Ansprüche 1 bis 7,
**dadurch gekennzeichnet, dass** die wenigstens eine bevorratete Substanz (S₁, S₂,...) aus den nachfolgenden Substanzen oder Substanzklassen gewählt ist: alkalisch oder saure Pufferlösung, den Natrium-, Kalium- und/oder Kalziumgehalt beeinflussende Substanzen, Blutverdünnende Substanzen, freie Radikalfänger, Glutamat, Aspartat, Herzrhythmus stabilisierende Substanzen (Lidocain), Leukozytenzahl, osmotisch aktive Substanzen, nämlich Salze, Glucose ,Proteine.

9. Vorrichtung nach einem der Ansprüche 1 bis 8,
**dadurch gekennzeichnet, dass** eine Monitoreinheit (M) vorgesehen ist, die mindestens ein Messmittel zur Erfassung wenigstens eines Parameters des Individuums aufweist, der aus der Gruppe der physiologischen Parameter des Individuums umfassend arteriellen Mitteldruck, zentralvenösen Druck, Pulmonalarterien-Druck, Sauerstoffsättigung und Bluttemperatur, ausgewählt ist, wobei die Monitoreinheit (M) mit der Auswerte- und Kontrolleinheit (A/K) zum Zwecke jeweils wenigstens eines einseitigen Informationsaustausches in Verbindung steht.

10. Vorrichtung nach einem der Ansprüche 1 bis 9,
**dadurch gekennzeichnet, dass** die Analyseeinheit (BA) eine die wenigstens eine Eigenschaft des Blutes des Individuums (P) erfassende Sensoreinheit (SEH) umfasst.

11. Vorrichtung nach Anspruch 10,
**dadurch gekennzeichnet, dass** die Sensoreinheit (SEH) mittel- oder unmittelbar mit dem Blutentnahmemittel (BE) verbunden ist.

12. Vorrichtung nach Anspruch 10 oder 11,
**dadurch gekennzeichnet, dass** die Sensoreinheit (SEH) zumindest einen der nachfolgenden Parameter des Blutes erfasst: pH-Wert, Sauerstoffpartialdruck, Kohlendioxidpartialdruck, Kaliumgehalt, Natriumgehalt, Calciumgehalt, Basenüberschuss, Lactatwert, Glucosegehalt.

13. Vorrichtung nach einem der Ansprüche 1 bis 12,
**dadurch gekennzeichnet, dass** die Analyseeinheit (A), die Wirkeinheit (KE) sowie die Auswerte- und Kontrolleinheit (A/K) als portable und einheitliche Baueinheit ausgeführt sind.

14. Vorrichtung nach Anspruch 10 und 13,
**dadurch gekennzeichnet, dass** die Sensoreinheit (SEH) Teil der einheitlichen Baueinheit ist.

15. Vorrichtung nach einem der Ansprüche 2 bis 14,
**dadurch gekennzeichnet, dass** längs des Blutleitungsweges (BL) zwischen dem Blutentnahmemittel (BE) und dem Rückführungsmittel (BR) dem Rückführungsmittel (BR) unmittelbar vorgeordnet eine Bypassleitung (A2) vorgesehen ist, durch die ein Teil des in das Individuum rückzuführenden modifizierten Blutes in die Analyseeinheit (BA) zuführbar ist, und dass die Dosiereinheit (D) im Falle eines abweichenden Soll-/Istwert-Vergleiches für wenigstens einen erfassbaren Blutparameter eine Änderung an der Mengenzugabe der wenigstens einen Substanz in den Blutleitungsweg (BL) vornimmt.

16. Vorrichtung nach einem der Ansprüche 1 bis 15,
**dadurch gekennzeichnet, dass** mittel- oder unmittelbar mit dem Rückführungsmittel (BR) verbunden eine Wärmetauschereinheit (WT) vorgesehen ist.

17. Vorrichtung nach Anspruch 16,
**dadurch gekennzeichnet, dass** die Auswerte- und Kontrolleinheit (A/K) Steuersignale (Si₁, Si₂, ...) generiert und an das Fördermittel (F) und die Wärmetauschereinheit (WT) derart abgibt, sodass Druck, Flussrate und Temperatur der in das Individuum zugeführten wenigstens einen Substanz wählbar sind.

18. Vorrichtung nach einem der Ansprüche 2 bis 17 ,
**dadurch gekennzeichnet, dass** längs des Blutleitungsweges (BL) zwischen dem Blutentnahmemittel (BE) und dem Rückführungsmittel (BR) eine Sauerstoffan- und -abreicherungseinheit für das Blut vorgesehen ist.

## Claims

1. A device for treating an individual having a cardiac arrest or cardiovascular arrest or a stroke, having a blood sampling means (BE) that can be applied to the individual (P) for sampling at least a portion of the blood from the individual, an analysis unit (BA), which is connected directly or indirectly to the blood sampling means for detecting and supplying at least one property of the blood in the form of a blood analysis result (BAE), an active unit (KE), which is connected directly or indirectly to a recirculation means (BR) that can be applied to the individual (P) and is designed for dispensing a substance to the individual by means of the recirculation means (BR),
**characterized in that**
the active unit (KE) comprises at least one reservoir unit (R) which stores at least two substances (S₁, S₂, ...), such that a dosing unit (D) which is combined with the reservoir unit (R) is provided, selecting at least one substance or preparing a mixture of at least two of the substances (S₁, S₂, ...) from the at least two substances (S₁, S₂, ...), at least taking into account one of the blood analysis results (BAE) determined by the analysis unit (BA), such that an evaluation and control unit (A/K) is provided, connected in a hard-wired or wireless manner for information exchange with the analysis unit (BA) and the dosing unit (D) and generating control signals (Si₁, Si₂, ...) for trigging the dosing unit (D) at least on the basis of the blood analysis result (BAE), such that the ischemic tissue damage, which would otherwise occur, can be prevented partially or completely by the at least one selected substance or the mixture after directly or indirectly introducing same through the recirculation means (BR) into the individual with an initial permeation of tissue areas with blood, said tissue areas having been uncoupled from the natural blood circulation system for either a short time or a long time.

2. The device according to Claim 1,
**characterized in that**
the blood sampling device (BE) that can be applied to the individual (P) is connected to the recirculation device (BR) which can be applied to the individual (P) by way of a blood line (BL); the analysis unit (BA) and the reservoir unit (R) and the dosing unit (D) are provided along the blood line pathway (BL); and the at least one selected substance or the mixture is miscible with the blood taken from the individual before being delivered to the individual via the recirculation line (BR) to obtain a modified blood.

3. The device according to Claim 2,
**characterized in that**
at least one delivery means (F) for an adjustable flow rate is/are provided along the blood line pathway (BL).

4. The device according to any one of Claims 1 to 3,
**characterized in that**
the analysis unit (BA) comprises a plurality of sensors (SE₁, ... SEₙ), each of which detects at least one blood parameter.

5. The device according to Claim 4,
**characterized in that**
the analysis unit (BA) detects sensorially at least one of the following blood parameters: pH, oxygen partial pressure (pO₂), carbon dioxide partial pressure (pCO₂), potassium content (K), sodium content (Na), calcium content (Ca), base excess (BE), lactate value (La), glucose content (Gu).

6. The device according to any one of Claims 1 to 5,
**characterized in that**
the reservoir unit (R) stores a number of substances (S₁, S₂, ...) which are present in solid, liquid or gaseous form and are stored in separate reservoir chambers which may be dosed individually or in combination by means of the dosing unit (D).

7. The device according to Claim 6,
**characterized in that**
the dosing unit (D) provides at least one mixing container (MB) which is connected to the individual reservoir chambers via individually controllable dosing means (V₁, V₂, ...) and the at least one mixing container (MB) is connected directly or indirectly to the recirculation means (BR) by means of another controllable dosing means.

8. The device according to any one of Claims 1 through 7,
**characterized in that**
the at least one substance (S₁, S₂, ...) that is stored is selected from the following substances or classes of substances: alkaline or acidic buffer solutions, substances that influence the sodium, potassium and/or calcium content, blood-diluting substances, free radical scavengers, glutamate, aspartate, heart-rhythm-stabilizing substances (lidocaine), leukocyte count, osmotically active substances, namely salts, glucose, proteins.

9. The device according to any one of Claims 1 to 8,
**characterized in that**
a monitor unit (M) is provided, having at least one measuring means for detecting at least one parameter of the individual, selected from the group of physiological parameters of the individual, comprising mean arterial pressure, central venous pressure, pulmonary arterial pressure, oxygen saturation and blood temperature, whereby the monitor unit (M) is connected to the evaluation and control unit (A/K) for the purpose of at least one unilateral information exchange.

10. The device according to any one of Claims 1 to 9,
**characterized in that**
the analysis unit (BA) comprises a sensor unit (SEH), which detects at least one property of the blood of the individual (P).

11. The device according to Claim 10,
**characterized in that**
the sensor unit (SEH) is connected directly or indirectly to the blood sampling device (BE).

12. The device according to Claim 10 or 11,
**characterized in that**
the sensor unit (SEH) detects at least one of the following parameters of the blood: pH, oxygen partial pressure, carbon dioxide partial pressure, potassium content, sodium content, calcium content, base excess, lactate value, glucose content.

13. The device according to any one of Claims 1 to 12,
**characterized in that**
the analysis unit (A), the active unit (KE) and the evaluation and control unit (A/K) are each embodied as a portable and uniform component.

14. The device according to Claims 10 and 13,
**characterized in that**
the sensor unit (SEH) is part of the uniform component.

15. The device according to any one of Claims 2 to 14,
**characterized in that**
a bypass line (A2) is provided immediately upstream from the recirculation means (BR) along the blood line path (BL) between the blood sampling site (BE) and the recirculation means (BR); a portion of the modified blood returning to the individual is returnable to the analysis unit (BA), and the dosing unit (D) makes a change in the quantitative addition of the at least one substance into the blood line pathway (BL) in the event of a deviating ideal/actual value comparison for the at least one detectable blood parameter.

16. The device according to any one of Claims 1 to 15,
**characterized in that**
a heat exchanger unit (WT) is provided, connected directly or indirectly to the recirculation means (BR).

17. The device according to Claim 16,
**characterized in that**
the evaluation and control unit (A/K) generates control signals (S₁, S₂, ...) and delivers them to the conveyance means (F), and the heat exchanger unit (WT) so that the pressure, flow rate and temperature of the at least one substance supplied to the individual can be selected.

18. The device according to any one of Claims 2 through 17,
**characterized in that**
an oxygen enrichment and depletion unit for the blood is provided along the blood line pathway (BL) between the blood sampling device (BE) and the recirculation means.

## Revendications

1. Dispositif de traitement d'un individu atteint d'un arrêt cardiaque ou circulatoire ou d'une attaque comportant un moyen de prise de sang (BE) applicable sur l'individu (P) pour prélever au moins une partie du sang de l'individu, une unité d'analyse (BA) reliée indirectement ou directement au moyen de prise de sang pour déterminer et fournir au moins une propriété du sang sous forme d'un résultat d'analyse de sang (BAE), d'une unité active (KE) qui est reliée indirectement ou directement à un moyen de renvoi (BR) applicable sur l'individu (P) et est conçue pour délivrer une substance à l'individu via le moyen de renvoi (BR),
**caractérisé en ce que** l'unité active (KE) comprend au moins une unité de réservoir (R) qui emmagasine au moins deux substances (S₁, S₂, ...), qu'il est prévu une unité de dosage (D) combinée à l'unité de réservoir (R) et qui, au moins en tenant compte d'un résultat d'analyse de sang (BAE) déterminé par l'unité d'analyse (BA), sélectionne parmi les au moins deux substances (S₁, S₂, ...) au moins une substance ou prépare un mélange d'au moins deux des substances (S₁, S₂, ...), étant prévue une unité d'exploitation et de contrôle (A/K) qui est en échange d'information filaire ou non filaire au moins avec l'unité d'analyse (BA) et l'unité de dosage (D) et, au moins sur la base du résultat d'analyse de sang (BAE), génère des signaux de commande (Si₁, Si₂, ...) pour commander l'unité de dosage (D) de manière à ce que, grâce à l'au moins une substance sélectionnée ou au mélange, après incorporation indirecte ou directe via le moyen de renvoi (BR) dans l'individu, lors d'une irrigation initiale des zones tissulaires dissociées pendant une courte ou longue durée du système cardiovasculaire naturel par du sang, les détériorations des tissus dues à l'ischémie se produisant autrement puissent être évitées partiellement ou complètement.

2. Dispositif selon la revendication 1,
**caractérisé en ce que** le moyen de prise de sang (BE) applicable sur l'individu (P) est relié via une voie de conduction sanguine (BL) au moyen de renvoi (BR) applicable sur l'individu (P), que l'unité d'analyse (BA) ainsi que l'unité de réservoir (R) et l'unité de dosage (D) sont prévues le long de la voie de conduction sanguine (BL) et que l'au moins une substance sélectionnée ou le mélange peut être mélangé avant délivrance à l'individu via le moyen de renvoi (BR) au sang prélevé sur l'individu pour obtenir un sang modifié.

3. Dispositif selon la revendication 2,
**caractérisé en ce que**, le long de la voie de conduction sanguine (BL), au moins un moyen de transport (F) est prévu pour un écoulement réglable.

4. Dispositif selon une des revendications 1 à 3,
**caractérisé en ce que** l'unité d'analyse (BA) comprend une multitude de capteurs (SE₁, SEₙ, ...) dont chacun détecte au moins un paramètre sanguin.

5. Dispositif selon la revendication 4,
**caractérisé en ce que** l'unité d'analyse (BA) détermine par détection au moins un des paramètres sanguins suivants : valeur pH, pression partielle d'oxygène (pO₂), pression partielle de dioxyde de carbone (pCO₂), teneur en potassium (K), teneur en sodium (Na), teneur en calcium (Ca), base en excès (BE), taux de lactate (La), teneur en glucose (Gu).

6. Dispositif selon une des revendications 1 à 5,
**caractérisé en ce que** l'unité de réservoir (R) emmagasine une multitude de substances (S₁, S₂, ...) qui sont présentes sous forme solide, liquide ou gazeuse et sont emmagasinées dans des cellules de réservoir séparées et peuvent être administrées isolément ou combinées au moyen de l'unité de dosage (D).

7. Dispositif selon la revendication 6,
**caractérisé en ce que** l'unité de dosage (D) prévoit au moins un récipient mélangeur (MB) qui est relié via des moyens de dosage (V₁, V₂, ...) pouvant être commandés individuellement aux cellules de réservoir individuelles et que l'au moins un récipient mélangeur (MB) est relié indirectement ou directement via un autre moyen de dosage pouvant être commandé au moyen de renvoi (BR).

8. Dispositif selon une des revendications 1 à 7,
**caractérisé en ce que** l'au moins une substance emmagasinée (S₁, S₂, ...) est sélectionnée parmi les substances ou classes de substances suivantes : solution tampon alcaline ou acide, substances jouant sur la teneur en sodium, potassium et/ou calcium, substances liquéfiant le sang, capteurs de radicaux libres, glutamate, aspartate, substances stabilisant le rythme cardiaque (lidocaïne), substances agissant par osmose sur le nombre de leucocytes, à savoir les sels, le glucose, les protéines.

9. Dispositif selon une des revendications 1 à 8,
**caractérisé en ce qu'**il est prévu une unité de contrôle (M) qui présente au moins un moyen de mesure pour déterminer au moins un paramètre de l'individu, lequel paramètre est sélectionné dans le groupe des paramètres physiologiques de l'individu comprenant la tension artérielle moyenne, la tension veineuse centrale, la tension artérielle pulmonaire, la saturation en oxygène et la température du sang, l'unité de gestion (M) étant en liaison avec l'unité d'exploitation et de contrôle (AK) aux fins de respectivement au moins un échange d'information unilatéral.

10. Dispositif selon une des revendications 1 à 9,
**caractérisé en ce que** l'unité d'analyse (BA) comprend une unité de détection (SEH) déterminant l'au moins une propriété du sang de l'individu (P).

11. Dispositif selon la revendication 10,
**caractérisé en ce que** l'unité de détection (SEH) est reliée indirectement ou directement au moyen de prise de sang (BE).

12. Dispositif selon la revendication 10 ou 11,
**caractérisé en ce que** l'unité de détection (SEH) détermine au moins un des paramètres suivants du sang : valeur pH, pression partielle d'oxygène, pression partielle de dioxyde de carbone, teneur en potassium, teneur en sodium, teneur en calcium, base en excès, taux de lactate, teneur en glucose.

13. Dispositif selon une des revendications 1 à 12,
**caractérisé en ce que** l'unité d'analyse (BA), l'unité active (KE), de même que l'unité d'exploitation et de contrôle (A/K) sont réalisées sous forme d'une unité de construction portable et unifiée.

14. Dispositif selon les revendications 10 et 13,
**caractérisé en ce que** l'unité de détection (SEH) fait partie de l'unité de construction unifiée.

15. Dispositif selon une des revendications 2 à 14,
**caractérisé en ce que**, le long de la voie de conduction sanguine (BL), entre le moyen de prise de sang (BE) et le moyen de renvoi (BR), juste en amont du moyen de renvoi (BR), il est prévu une conduite de dérivation (A2)) à travers laquelle une partie du sang modifié à renvoyer dans l'individu peut être acheminée dans l'unité d'analyse (BA) et que l'unité de dosage (D), dans le cas d'une comparaison valeur théorique/réelle divergente pour au moins un paramètre sanguin détectable, procède à une modification d'apport quantitatif de l'au moins une substance dans la voie de conduction sanguine (BL).

16. Dispositif selon une des revendications 1 à 15,
**caractérisé en ce qu'**il est prévu une unité d'échange de chaleur (WT) en liaison indirecte ou directe avec le moyen de renvoi (BR).

17. Dispositif selon la revendication 16,
**caractérisé en ce que** l'unité d'exploitation et de contrôle (A/K) génère des signaux de commande (Si₁, Si₂, ...) et les délivre au moyen de transport (F) et à l'unité d'échange de chaleur (WT) de manière à ce que la pression, la vitesse d'écoulement et la température de l'au moins une substance apportée à l'individu soient sélectionnables.

18. Dispositif selon une des revendications 2 à 17,
**caractérisé en ce que**, le long de la voie de conduction sanguine (BL), entre le moyen de prise de sang (BE) et le moyen de renvoi (BR), une unité d'enrichissement et d'appauvrissement en oxygène pour le sang est prévue.
